(19) Europäisches Patentamt
European Patent Office
Office européen des brevets

(11) Publication number: **0 183 083**
**A1**

# (12) EUROPEAN PATENT APPLICATION

(21) Application number: **85113918.8**

(22) Date of filing: **31.10.85**

(51) Int. Cl.[4]: **C 07 C 13/70**
C 07 C 1/32, C 07 C 87/30

(30) Priority: **02.11.84 IT 2342484**

(43) Date of publication of application:
**04.06.86 Bulletin 86/23**

(84) Designated Contracting States:
**AT BE CH DE FR GB LI NL SE**

(71) Applicant: **Montedison S.p.A.**
**31, Foro Buonaparte**
**I-20121 Milan(IT)**

(72) Inventor: **Malacrida, Alessandro**
**49, via Lambro**
**I-20050 Sovico Milan(IT)**

(72) Inventor: **Montanari, Fernando**
**29, via Muratori**
**Milan(IT)**

(72) Inventor: **Bottaccio, Giorgio, Dr.**
**16, via Manin**
**Novara(IT)**

(72) Inventor: **Campolmi, Stefano, Dr.**
**27, via Andrea Costa**
**Novara(IT)**

(74) Representative: **Weinhold, Peter, Dr. et al,**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8**
**D-8000 München 40(DE)**

## (54) Process for the preparation of (2,2)-paracyclophane.

(57) Process for the preparation of (2,2)-paracyclophane starting from p.methyl-benzyl-trimethyl-ammonium halide, consisting in first preparing the p.methyl-benzyl-trimethyl-ammonium hydroxide and successively subjecting the product thus obtained to the HOFMANN Elimination in the presence of a biphasic system consisting of an inert organic solvent and an alkaline solution having a concentration of at least 20% by weight.

The present invention refers to a process for the preparation of (2,2)-paracyclophane of the general formula:

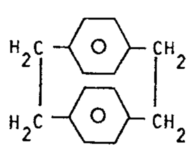

(I)

More particularly, the present invention relates to a process for the preparation of (2,2)-paracyclophane of formula (I), starting from a p.methyl-benzyl-trimethyl-ammonium halide of the formula:

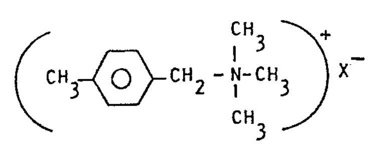

(II)

wherein: X is a halide such as chlorine, bromine, etc., by the elimination of HOFMANN.

EP 0 183 083 A1

BACKGROUND OF THE INVENTION

The (2,2)-paracyclophane is a well known product and is mainly used as an intermediate in the preparation of polyp.xylilene, a polymer of high performances with regard to its capacity for coating surfaces.

There have been suggested various different procedures for preparing the (2,2)-paracyclophane (I). Said known processes or procedures are not, however, altogether satisfactory and do not lend themselves to practical application on an industrial scale.

Thus, for instance, in "Organic Synthesis-Collective", Volume 5, Hohn Whiley & Jons Inc., New York, London, Sydney, Toronto, 1973, pp. 883-886, there is described a process for producing (2,2)-paracyclophane through the HOFMANN elimination, starting from p.methyl-benzyl-trimethyl-ammonium hydroxide by the reaction of the corresponding bromide with silver oxide. The elimination is conducted in the presence of an inert organic solvent (toluene) and gives a yield of 10-11%, with the formation of undesired polymeric by-products.

In order to improve the yield in the above-cited process, in the European Patent Application No. 108,297, it is suggested that the HOFMANN elimination be carried out starting from compound (II) in the presence of a base consisting of a sodium or potassium hydroxide and of dimethylsulphoxide. This process allows to obtain high yields (around 70% by weight). However, the recovery of the dimethylsulphoxide makes the process relatively unattractive for an industrial application. Moreover, the (2,2)-paracyclophane obtained by this process is not altogether satisfactory as far as its purity is concerned.

THE PRESENT INVENTION



Thus, an object of the present invention is that of providing a process for the preparation of (2,2)-paracyclophane of formula (I) that is free of the above-mentioned drawbacks.

More particularly, an object of the present invention is to provide a simple and cheap process that results in (2,2)-paracyclophane of formula (I) in the pure form, at industrially acceptable yields.

We have now found that these and other objects can be achieved by a process for the preparation of p.methyl-benzyl-trimethyl-ammonium halide of formula (II), that consists in the following separate steps carried out in the given order:

(a) preparation of p.methyl-benzyl-trimethyl-ammonium hydroxide, and

(b) a HOFMANN elimination reaction carried out on the p-methyl-benzyl-trimethyl-ammonium hydroxide in the presence of a biphasic system consisting of an inert organic solvent and of an aqueous solution of an alkaline hydroxide having a concentration of at least 20% by weight.

It is expressly pointed out that the two above reported procedural steps must be conducted in a strictly separate way and that the Hofmann elimination must be carried out on p.methyl-benzyl-trimethyl-ammonium hydroxide essentially free of halogen ions. In fact, our tests have evidenced quite clearly that the presence of halogen ions negatively influences the Hofmann elimination reaction, lowering its yield.

The p.methyl-benzyl-trimethyl-ammonium hydroxide may be prepared starting from the corresponding halide according to any known process. In practice, it is preferable to prepare th

p.methyl-benzyl-trimethyl-ammonium hydroxide by eluting an aqueous solution of a corresponding halide through a column filled with a basic ion-exchanging resin.

The Hofmann elimination is conducted on the p-methyl-benzyl-trimethyl-ammonium hydroxide obtained in the first step or stage of the process, at a temperature comprised between 20° and 130°C, and for reaction times comprised between 10 and 70 hours, in the presence of a biphasic system consisting of an organic solvent and of an aqueous solution of an alkaline hydroxide having a concentration exceeding 20% by weight, but preferably comprised between 30% and 50% by weight.

Preferred reaction times are comprised between 20 and 40 hours while the preferred temperatures are from 60° to 100°C, extremes included.

Any inert organic solvent may be used in practicing the present process. Among these, the following are preferred; aromatic hydrocarbons such as toluene, xylene, benzene, etc.

At the end of the elimination reaction, the product formed can be separated according to known techniques that are substantially of conventional type.

The process, object of the present invention, allows to obtain (2,2)-paracyclophane of a high degree of purity, at industrially acceptable yields.

The following examples are given to illustrate the invention in more detail and are not intended to be limiting.

In all the examples given, where not specifically indicated otherwise, the percentages and ratios are by weight.

EXAMPLE 1 (Comparative)

An aqueous solution consisting of 150 g of p.methyl-benzyl-trimethyl-ammonium chloride in 90 ml of water, was slowly added dropwise and under stirring, in a period of one hour, to a biphasic system maintained at a temperature of 90°C and consisting of a solution of 210 g of NaOH in 240 ml of water and of 3 liters of toluene. Stirring and heating at 90°C were carried out for 40 hours.

The reaction mixture was then filtered, the (water/toluene) phases were separated, the organic phase was washed with water, concentrated and finally cooled down. Thereby, by precipitation, there were obtained 15.5 g of (2,2)-paracyclophane (with a yield of 20% by weight), showing a melt point of between 280° and 282°C.

EXAMPLE 2 (Comparative)

To 25 ml of an aqueous solution 2 M of p.methyl-benzyl-trimethyl-ammonium hydroxide (density = 1.03 g/ml) were added 200 ml of toluene and 2 g of NaOH, corresponding to 10% by weight.

The system was allowed to react under constant stirring at 90°C, for 40 hours. Thereafter, the phases were separated and the organic phase was washed with water and then evaporated to dryness. Only traces of (2,2)-paracyclophane were found.

## EXAMPLE 3

(A) Preparation of the p.methyl-benzyl-trimethyl-ammonium hydroxide:

20 g of p.methyl-benzyl-trimethyl-ammonium chloride dissolved in 300 ml of water were eluted in 3 hours through a column filled with a strong ion-exchanging basic resin, DOWEX 1-X8 (50-100 mesh), said resin having preliminarily been converted to the hydroxide-ion form by means of a treatment with a 3 M solution of NaOH. For the exchange there was used a volume of humid (moist) resin equal to 350 ml.

(B) To 125 ml of an aqueous solution 0.4 M of p.methyl-benzyl-trimethyl-ammonium hydroxide, as prepared above, were admixed carefully and under stirring 125 ml of xylene and 78 g of NaOH, corresponding to 40% by weight. This reaction mixture was then kept under stirring for 50 hours at 60°-65°C. Thereupon, the reaction mass was refluxed in order to solubilize all the (2,2)-paracyclophane that had been formed. The mass was then filtered in order to separate the polymer; the phases were separated and the organic phase was washed with water and then concentrated.

By refrigeration, there precipitated 2.15 g of (2,2)-paracyclophane (with a yield of 40% by weight) which showed a melt point comprised between 283° and 286°C.

## EXAMPLE 4

p.methyl-benzyl-trimethyl-ammonium hydroxide was prepared according to the procedures described in Example 3A.

Following the operational steps indicated in Example 3B, to 125 ml of an aqueous solution 0.4 M of p.methyl-benzyl-trimethyl-ammonium hydroxide, there were added 125 ml of zylene and 130 ml of KOH (minimum titer: 85%).

After 40 hours of heating at 60°-65°C, there were obtained 2.2 g of (2,2)-paracyclophane with a melt point comprised between 283° and 286°C.

What is claimed:

1. Process for the preparation of (2,2)-paracyclophane, starting from a p.methyl-benzyl-trimethyl-ammonium halide, by means of a HOFMANN elimination reaction, characterized in that said process consists of the following steps or phases carried out separately and in the given order of succession:

(a) preparation of the p.methyl-benzyl-trimethyl-ammonium hydroxide, and

(b) a HOFMANN elimination carried out on the p.methyl-benzyl-trimethyl-ammonium hydroxide, in the presence of a biphasic system consisting of an inert organic solvent and of an aqueous solution of an alkaline hydroxide having a concentration of at least 20% by weight.

2. Process according to claim 1, characterized in that the concentration of the aqueous solution of an alkaline hydroxide is comprised between 30% and 50% by weight.

3. Process according to one or both of the claims 1-2, characterized in that the alkaline hydroxide is selected from the group consisting of sodium hydroxide and potassium hydroxide.

4. Process according to one or more of the claims 1-3, characterized in that the inert organic solvent is an aromatic hydrocarbon.

5. Process according to one or more of the claims 1-4, characterized in that the HOFMANN elimination is carried out at a temperature comprised between 20° and 130°C and for a reaction time comprised between 10 and 70 hours.

6. Process according to one or more of the claims 1-5, characterized in that the p.methyl-benzyl-trimethyl-ammonium hydroxide is prepared by elution of an aqueous solution of a corresponding halide by passing said solution through a column filled with a basic ion-exchanging resin.

European Patent Office

0183083

Application number

# EUROPEAN SEARCH REPORT

EP 85 11 3918

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,X | EP-A-0 108 297 (MERCK) * Page 2, line 1 - page 3, line 11 * | 1-5 | C 07 C 13/70<br>C 07 C 1/32<br>C 07 C 87/30 |
| X | BULLETIN OF THE CHEMICAL SOCIETY OF JAPAN, vol. 46, November 1973, pages 3519-3530, Tokyo, JP; T. OTSUBO et al.: "Layered compounds. XV. Synthesis and properties of multilayered cyclophanes" * Page 3520, paragraph one of "Results and Discussion"; page 3526, last paragraph - page 3527, first paragraph * | 3,4,6 | |
| | | | **TECHNICAL FIELDS SEARCHED (Int. Cl.4)** |
| | | | C 07 C 13/00<br>C 07 C 1/00 |

The present search report has been drawn up for all claims

| Place of search | Date of completion of the search | Examiner |
|---|---|---|
| THE HAGUE | 21-03-1986 | VAN GEYT J.J.A. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document